# EUROPEAN PATENT SPECIFICATION

(11) **EP 2 470 286 B1**
(45) Date of publication and mention of the grant of the patent: **14.05.2014**
(21) Application number: 10828434.0
(22) Date of filing: 30.08.2010
(51) Int. Cl.: B01D 46/00, A61L 9/16

(54) **AIR CLEANING FILTER COMPRISING KIMCHI LACTIC ACID BACTERIA AND DISINFECTANT AND PROCESS FOR PRODUCING THE SAME**
LUFTREINIGUNGSFILTER MIT KIMCHI-MILCHSÄUREBAKTERIEN UND DESINFEKTIONSMITTEL SOWIE VERFAHREN ZU SEINER HERSTELLUNG
FILTRE POUR LE NETTOYAGE DE L'AIR COMPRENANT UNE BACTÉRIE D'ACIDE LACTIQUE DE KIMCHI ET UN DÉSINFECTANT, ET SON PROCÉDÉ DE FABRICATION

(30) Priority: 03.11.2009 KR 20090105346
(43) Date of publication of application: 04.07.2012
(73) Proprietor: LG Electronics Inc., Seoul 150-721 (KR)
(72) Inventor: LEE, Sung Hwa, Changwon-si Gyeongsangnam-do 641-711 (KR)
(74) Representative: Vossius & Partner
(86) International application number: PCT/KR2010/005831
(87) International publication number: WO 2011/055898

(56) References cited:
- EP-A2- 1 790 914
- WO-A1-2011/055893
- WO-A2-2007/058475
- WO-A2-2007/058476
- WO-A2-2007/058476
- WO-A2-2007/061238
- KR-A- 20070 099 799
- KR-A- 20070 099 806
- KR-A- 20070 099 807
- DATABASE WPI Week 200756 24 May 2007 (2007-05-24) Thomson Scientific, London, GB; AN 2007-586580 XP002694115, & WO 2007/058476 A2 (LG ELECTRONICS INC) 24 May 2007 (2007-05-24)

## Description

### Technical Field

The present invention relates to an air cleaning filter comprising kimchi lactic acid bacteria, and more particularly the present invention relates to an air cleaning filter which comprises a carrier coated with kimchi lactic acid bacteria and disinfectant for making effective removal or sterilization of microbes, such as bacteria, fungi, and virus from the air, and a process for producing the same. The present invention relates to an air cleaning filter which comprises a carrier coated with kimchi lactic acid bacteria, disinfectant and fermented *Ecklonia cava* extract, and a process for producing the same.

### Background Art

Currently, as interest in the environment is increasing, demand for cleaning the room air is also increasing. Consequently, various air cleaning devices for removing contaminants from the air have been developed and are under development. An air cleaning filter device uses an air cleaning filter which is required to be in various shapes and have various characteristics according to the types and sizes of objects to be removed; and the characteristics of the objects to be removed, and thus, a variety of filters are under development.

Especially, an air cleaning filter is required to have anti-microorganism or bactericidal capability for adequate removal or disinfection of microbes, such as bacteria, fungi, and virus floating in the air in order to obtain a satisfactory air cleaning effect.

On the other hand, it has been studied that the virus, a micro life which can not survive for itself but requires a host essentially, infects the host cell, parasitizes at the host, and reproduces itself according to its genetic information in a large amount. That means that the virus is different from other lives and has either a DNA or RNA at a nucleic acid in a cell. The typical examples of RNA viruses are an influenza virus, Ebola virus, AIDS virus, and so on. Since it is known that the RNA viruses make mutation 100,000 to 10,000,000 times easier than the DNA viruses, the prophylaxis of the infection of these RNA viruses is very difficult. Particularly, the influenza A (H1N1) virus, generally called as'a new kind of influenza'has a new H1N1 type genome structure different from an existing epidemic influenza virus because it is, for example, born a new kind of virus of swine influenza due to a human influenza virus adapted to a swine host and mixed with an already existing swine influenza virus.

If the new kind of influenza virus would be combined with an avian influenza virus in the swine host by means of gene swapping and thus generate another new kind of virus, then the global infection could be caused by it. It is, also, reported that a human being could also be infected with the above new kind of virus, and even a human being to a human being infection would be very easy, too. With regard to the viral infection, it is also known that the kimchi lactic acid bacteria have a function to cut off a viral infection passage to a human body cell to suppress the virus.

Meanwhile, it also has been studied that a Korean traditional food kimchi, prepared from many kinds of materials, enables to maintain a balance of nutrients. Kimchi is also known for its antibacterial activity, anti-inflammatory, and even anti-cancerous activity, since it comprises many kinds of antibiotics including allicin and a large amount of lactic acid bacteria influencing to metabolic activity of intestinal flora.

Moreover, according to the result of recent studies, since the kimchi lactic acid bacteria is effective for treatment of viral disease, such as the avian influenza and the new kind of influenza A (H1N1 virus), interest in Koreans traditional food kimchi becomes higher and higher.
EP 1 790 914 A2 relates to an air conditioner including a main body formed by a plurality of members wherein at least one of the members includes a Kimchi lactic bacterium zymogenic material.
WO 2011/055893 A1 relates to an air cleaning filter including a carrier provided therein including a protein deactivating agent coated thereon for removing or sterilizing bacteria, fungi, or virus in the air.
WO 2007/058476 A2 relates to an article with an antimicrobial property, and a manufacturing method thereof.
WO 2007/058475 A2 relates to a molded article with an antimicrobial property, and a manufacturing method thereof.
WO 2007/061238 A2 relates to a refrigerator including a refrigerating cycle having an evaporator, a space for exchanging heat with the evaporator, a region for exchanging heat between the evaporator and the space and a storage chamber and a door for defining the space.

### Disclosure of Invention

### Technical Problem

An object of the present invention is to provide an air cleaning filter in which kimchi lactic acid bacteria and a disinfectant are used for removal or sterilization of microbes, such as bacteria, fungi, and virus from the air. An object of the present invention is to provide an air cleaning filter in which kimchi lactic acid bacteria, a disinfectant and fermented *Ecklonia cava* extracts are used for removal or sterilization of microbes, such as bacteria, fungi, and virus from the air.

Another object of the present invention is to provide a process for producing an air cleaning filter comprising a carrier coated with kimchi lactic acid bacteria and a disinfectant for removal or sterilization of microbes, such as bacteria, fungi, and virus from the air. Another object of the present invention is to provide a process for producing an air cleaning filter comprising a carrier coated with kimchi lactic acid bacteria, a disinfectant and fermented *Ecklonia cava* extract for removal or sterilization of microbes, such as bacteria, fungi, and virus from the air.

### Solution to Problem

To achieve these objects and other advantages and in accordance with the purpose of the invention, as embodied and broadly described herein, an air cleaning filter comprises a carrier coated with kimchi lactic acid bacteria, a disinfectant, and fermented *Ecklonia cava* extract.

In another aspect of the present invention, a process for producing an air cleaning filter includes the step of coating and immobilizing kimchi lactic acid bacteria, disinfectant, and, fermented *Ecklonia cava* extract on a carrier. Alternatively, the process for producing an air cleaning filter according to the present invention includes the step of coating a coating solution on the carrier, the coating solution including the kimchi lactic acid bacteria, the disinfectant, the fermented *Ecklonia cava* extract, and a binder selected from a group including silicon modified acryl resin, silicon modified epoxy resin, urethane resin, acryl resin, and silicon resin; and the step of drying the carrier coated thus.

As a material of the carrier in the air cleaning filter of the present invention, as far as the material carries out its role of air cleaning, any one can be used regardless of kinds, shapes, sizes, and producing methods, without any limitation. For an example, glass fiber, such as ion exchange fiber, cellulose fiber, asbestos fiber; various kinds of organic fibers; and various kinds of inorganic fibers can be used. Also, metal, such as zinc, copper, aluminum; or even plastic can be used. These materials can be used for various purposes according to the properties of the materials.

A shape of the carrier used in the air cleaning filter according to the present invention can also be modified appropriately, such as a honey comb shape, a granule shape, a net shape, a filter paper shape, a cotton shape, a mesh shape, a plate shape, a foam shape, and the like, according to the air cleaning devices to which the carrier is applied, without particular limitation.

The air cleaning filter according to the present invention can be used as, or together with, deodorant filter, such as an activated charcoal filter used in domestic appliance, such as a refrigerator and an air conditioner, HEPA (high efficiency particulate air) filter, and a filter in an air cleaner of a car.

The kimchi lactic acid bacteria used in the present invention may be extracted from kimchi directly, or commercially available without any limitation, regardless of state. For an example, the kimchi lactic acid bacteria can be in the state of any one selected from a group of a Kimchi lactic acid bacteria culture solution, a concentration of the culture solution, an extract of the culture solution, a dried culture solution thereof, and a mixture thereof.

Preferably, the kimchi lactic acid bacteria can be any one selected from a group including *Leuconostoc* genus Kimchi lactic acid bacteria, *Lactobacillus* genus Kimchi lactic acid bacteria, *Weissella* genus Kimchi lactic acid bacteria and a mixture thereof. It is particularly preferable that Kimchi lactic acid bacteria are *Leuconostoc* genus. It is preferable that *Leuconostoc* genus Kimchi lactic acid bacteria are any one selected from a group including *Leuconostoc citreum, Leuconostoc lactis, Leuconostoc mesenteroides subsp. dextranicum, Leuconostoc mesenteroides subsp. Mesenteroides, Leuconostoc argentinum, Leuconostoc carnosum, Leuconostoc gellidum, Leuconostoc kimchii, Leuconostoc inhae, Leuconostoc gasicomitatum,* and a mixture thereof. It is preferable that the *Lactobacillus* genus Kimchi lactic acid bacteria are any one selected from a group including *Lactobacillus brevis, Lactobacillus acidophilus, Lactobacillus bulgaricus, Lactobacillus plantarum, Lactobacillus kimchii, Lactobacillus plantarum, Lactobacillus curvatus subsp. curvatus, Lactobacillus sakei sibsp. sakei* and a mixture thereof. It is also preferable that the *Weissella* genus Kimchi lactic acid bacteria are any one selected from *Weissella koreensis, Weissella hanii, Weissella kimchii, Weissella soli, Weissella confusa,* and a mixture thereof.

It is preferable that kimchi lactic acid bacteria used in the present invention are in a form of a lactic acid bacteria culture extract.

As disinfectant used in the present invention having antibacterial, antifungal and antiviral activity, any form and/or kind of disinfectant can be used without particular limitation as far as the disinfectant is harmless to a human body. It is preferable that disinfectant is any one selected from a group including sodium desoxycholate, glutaldehyde and quaternary ammonium, and more preferably, glutaldehyde. Sodium desoxycholate has an effect in which sodium desoxycholate gives influences to a cell membrane to suppress cell growth, and glutaldehyde has an effect in which glutaldehyde connects between proteins to set enzymes, thereby making activity of the protein poor. Quarternary ammonium raises pH, and thus, makes a living condition of the bacteria unfavorable.

The air cleaning filter according to the present invention further includes fermented *Ecklonia cava* extract. Fermented *Ecklonia cava* extract includes dieckol component of a phlorotannin group which has an excellent antibacterial/anti-inflammatory activity and has been used for a long time. In the present invention, after mixing *Ecklonia cava* and distilled water, the mixture is pulverized with a homogenizer, sterilized by a steam high pressure sterilizer at 121°C for 15 minutes, left at a room temperature until cooled down, and fermented in a shake culture at 30°C. Then, methanol is added to the *Ecklonia cava* fermented thus, stirred and extracted in a mantle for 3 hours for three times repeatedly, filtered, and concentrated under vacuum in a 60°C isothermal water tank with a rotary vacuum vaporizer, and an extract component thereof is vaporized to the maximum, to obtain the fermented *Ecklonia cava* extract powder.

The air cleaning filter of the present invention is produced by a process including the step of coating and immobilizing kimchi lactic acid bacteria and disinfectant on a carrier. If necessary, the air cleaning filter of the present invention is produced by a process including the step of coating and immobilizing the kimchi lactic acid bacteria, the disinfectant and the fermented *Ecklonia cava* extract on the carrier, at a time or in succession.

The air cleaning filter of the present invention can be produced by a process including the step of spraying a solution (coating solution) to a carrier or dipping the carrier in the coating solution including the kimchi lactic acid bacteria, the disinfectant, fermented *Ecklonia cava* extract, and a binder selected from a group including silicon modified acryl resin, silicon modified epoxy resin, urethane resin, acryl resin, and silicon resin. Optionally, the coating solution can further include metal. The metal can be Ag, Cu and Zn, and can be used individually, or as mixture thereof, preferably with about 1v% to 5v%.

The air cleaning filter according to the present invention can be produced by coating kimchi lactic acid bacteria and disinfectant, or kimchi lactic acid bacteria, disinfectant and a fermented *Ecklonia cava* extract on a carrier directly. Or, the air cleaning filter according to the present invention can be produced by coating a coating solution on a carrier by spraying or dipping after preparing the coating solution. The step of coating and immobilizing the kimchi lactic acid bacteria and disinfectant, or kimchi lactic acid bacteria, disinfectant, and fermented *Ecklonia cava* extract on a carrier in the air cleaning filter can be performed by a method known in this field of art. Depending on cases, it is required to change the kimchi lactic acid bacteria and disinfectant, or kimchi lactic acid bacteria, disinfectant and fermented *Ecklonia cava* extract to a state suitable for coating depending on properties of the carrier. In order to immobilize the kimchi lactic acid bacteria and disinfectant, and optionally fermented *Ecklonia cava* extract on the carrier, a chemical or physical immobilizing technology suitable for the purpose of use can be used.

In another method for producing an air cleaning filter in which the kimchi lactic acid bacteria, disinfectant and, fermented *Ecklonia cava* extract is coated on a carrier, a coating solution is prepared by mixing the kimchi lactic acid bacteria, disinfectant and, fermented *Ecklonia cava* extract with a binder, such as silicon modified acryl resin, silicon modified epoxy resin, urethane resin, acryl resin and silicon resin, and the coating solution prepared thus is coated on a surface of the carrier in an air cleaning filter by spraying or dipping. There is no particular limitation in the method for preparing the coating solution as far as the kimchi lactic acid bacteria, disinfectant and, fermented *Ecklonia cava* extract can be mixed with the binder enough to be coated on the surface of the carrier. It is preferable that a coating solution consists of 3v% to 10v% of kimchi lactic acid bacteria, 0.05v% to 3v% of disinfectant, 0.5v% to 10v% of fermented *Ecklonia cava* extract, and 85v% to 95v% of the binder and water in view of mixing and coating. In this instance, the binder and water has 20v% to 30v% of the binder and 70v% to 80v% of the water. In above composition, if a ratio thereof is too high, coating is difficult, and if too low, a performance can be poor. Depending on cases, metal may be further added. The metal can be Ag, Cu and Zn, and can be used individually, or mixed, and an amount of the metal can be about 1v% to 5v% in the composition.

In the producing method of the present invention, before the step for coating with kimchi lactic acid bacteria, disinfectant, and fermented *Ecklonia cava* extract and the like on a carrier, the step for washing the carrier to be used with appropriate washing water or drying the carrier washed thus by heat treatment can be added. Depending on cases, it is preferable that oil stuck to a surface of the carrier of metal or the like during production or storage is removed therefrom. In a case the carrier is dried, a drying period and temperature can be adjusted according to a shape, a kind and a size of the carrier to be used. Moreover, after coating kimchi lactic acid bacteria, disinfectant, and fermented *Ecklonia cava* extract on the carrier, the step for drying the carrier coated thus can be included.

The air cleaning filter produced according to the present invention is cut to a required size for use as the air cleaning filter of an air cleaning device. The air cleaning filter produced according to the present invention can be used, not only individually within the same product, but also together with a related art air cleaning filter, deodoring filter, and the like. Thus, the air cleaning filter of the present invention can be used widely, in domestic or office air cleaning devices where the air cleaning filter is required, and in automobiles, refrigerators, air conditioners, and other domestic appliances.

### Advantageous Effects of Invention

The present invention has following advantageous effects.

The air cleaning filter according to the present invention having the kimchi lactic acid bacteria, disinfectant, and fermented *Ecklonia cava* extract coated thereon can effectively clean up microbes, such as bacteria, fungi, and virus from the air by disinfecting and sterilizing the microbes. Especially, the air cleaning filter according to the present invention can provide a safe and efficacious antiviral effect against the avian flu virus, the human influenza virus, and the new kind of influenza virus owing to the kimchi lactic acid bacteria and the fermented *Ecklonia cava* extract.

### Best Mode for Carrying out the Invention

Reference will now be made in detail to the specific embodiments of the present invention, examples of which are illustrated in the accompanying drawings. Wherever possible, the same reference numbers will be used throughout the drawings to refer to the same or like parts.

### 1. Production of an air cleaning filter

After mixing 30g of *Ecklonia cava* with 600ml of distilled water, the mixture is pulverized with a homogenizer, sterilized by a steam high pressure sterilizer at 121°C for 15 minutes, left at a room temperature until the mixture is cooled down, and fermented for 3 days in a shake culture at 30°C. Then, 6 liters of methanol is added to the *Ecklonia cava* fermented thus, stirred and extracted in a mantle for 3 hours for three times repeatedly, filtered, and concentrated under vacuum in a 60°C isothermal water tank with a rotary vacuum vaporizer, and an extract component thereof is vaporized to the maximum, to obtain 24g of fermented *Ecklonia cava* extract powder.

Coating solutions are prepared each to include the fermented *Ecklonia cava* extract; *Leuconostoc citreum,* which is kimchi lactic acid bacteria culture solution extract commercially available; glutaldehyde; metal in which Ag, Cu, and Zn form a metal sol at a ratio of 1:1:2; silicone modified epoxy resin binder comprising 10% epoxy resin and 90% silicone resin; and distilled water, according to sets of the composition in the following table 1.

Units in the table 1 are v%.

**Table 1**

| | 1* | 2* | 3* | Metal | Binder | Distilled water |
|---|---|---|---|---|---|---|
| Embodiment 1 | 5 | 1 | - | 1 | 25 | 68 |
| Embodiment 2 | 5 | 1 | 2 | 1 | 25 | 68 |

| | | | | | | |
|---|---|---|---|---|---|---|
| 1*: Kimchi lactic acid bacteria culture solution extract 2*: Glutaldehyde 3*: Fermented *Ecklonia cava* extract | | | | | | |

A Jabra type filters of an electrostatic non woven fabric is dipped in each of the coating solutions of above sets of the composition for two minutes, respectively. The filters dipped thus are removed from the coating solutions, left at a room temperature for 30 to 60 minutes (to let the coating solutions flow down), and dried at 70°C for 60 minutes to fabricate filter samples, respectively.

### 2. An antibacterial test of the filter against Staphylococcus aureus

### (1) Preparation of a test piece

A pre-incubated *S*. *aureus* solution (10⁹ cfu/ml) is diluted and inoculated to a 100 ml of sterilized neutral solution (0.2% culture broth, 0.5% NaCl) in a conical flask so as to be 10⁵ cfu/ml.

- 30 test samples each with 1.0x1.0cm square are prepared, and the obtained neutral solution above is placed in the test samples. These are called as anti-bacteria processed test pieces.

- 30 filters which are not subjected to any anti-bacteria process are provided with sizes the same with the test samples, respectively, and placed in 100ml of the neutral solution above in order to provide the control group.

- As the control group, after diluting a flask prepared by using a physiological salt solution and shaking well, 1 ml of the obtained solution is taken and an initial number of bacteria is counted by a mixed dilution plating incubation method (pour plate).

- The filter and the conical flask, having the S. *aureus* inoculated thereto thus and shaken at 35°C shaking incubator at 150 rpm, are made to react for 24 hours.

- The *S. aureus* made to react with the filter for 24 hours are diluted in decimal notation, 1 ml of diluted solution thereof is placed in a sterilized plate, and is incubated in a mixed dilution plate by using nutrient agar.

- The bacterial plate is incubated at a 37°C incubator for 18 to 24 hours.

### (2) Test result

After 24 hours, the colonies of the *S. aureus* are counted and the following table 2 shows a result of the count.

**[49] Table 2**

| Antibacterial performance on S. aureus | | | |
|---|---|---|---|
| | | Time (hours) | |
| | | 0 | 24 |
| Control group | No. of bacteria | 2.2x10⁴ | 1.1x10⁹ |
| | Reduction ratio | | |
| Embodiment 1 | No. of bacteria | 2.2x10⁴ | 7.7x10² |
| | Reduction ratio | | 99.9% |
| Embodiment 2 | No. of bacteria | 2.2x10⁴ | 1.6x10³ |
| | Reduction ratio | | 99.9% |

### 3. Anti-fungus rate test of the filters on Aspergillus niger

In order to determine an antifungal effect of the filters, anti-fungus rate tests are performed for the filters of embodiments 1 to 2 according to JIS Z 2911 (Antifungal test method).

As a result of the tests, both embodiments 1 and 2 show"0". In this instance,"0"denotes no mycelia growth,"1"denotes mycelia growth being less than 1/3 of an entire area, and"2"denotes mycelia growth being more than 1/3 of an entire area.

### 4. Antiviral test (Shaking flask method) of the filters on Feline calicivirus (FCV)

### (1) Preparation of a test piece

- 6 test samples are provided, each with a size of 1.0x1.0cm square, and placed on maintenance media {2% FBS (Fetal Bovine Serum), DMEM (Dulbeco's Modified Eagle's Medium)}, respectively.

- FCV diluted to about 10⁵ TCID₅₀/ml is inoculated to the test samples, and reacted at a room temperature for predetermined time periods, respectively.

- When the reaction is finished, the virus is diluted at maintenance media (2% FBS, DMEM) in decimal notation.

- After removing a growth medium by using an aspirator in a 96 well-plate of single film, the obtained diluted virus is inoculated to 8 wells each by 25µl.

- After adsorbing the virus in 5% CO₂ incubator for 90 minutes at 37°C, 100ml of maintenance medium is added to each of the wells.

- A potency of the virus is determined by making the virus to react with the extract for a predetermined time period, comparing an amount of the virus remained after the reaction to the control group, and calculating TCID₅₀ (tissue culture infectious dose 50) measured from CrFK (Crandel feline kidney) cells.

### (2) Result of test

After visualizing cells dissolved by the virus on a fifth day of virus culture, dilution stage of the well showing more than 50% of CPE is calculated by Reed-Munch method and expressed with Log TCID₅₀. The following table 3 shows a result of the calculation.

**Table 3**

| Antiviral performance on FCV | | | | |
|---|---|---|---|---|
| | | Time (hours) | | |
| | | 5 | 8 | 10 |
| Control group | | 5.01.0x10⁵ | 4.53.2x10⁴ | 3.53.2x10³ |
| Embodiment 1 | TCID₅₀ | 2.171.5x10² | 2.171.5x10² | 2.01.0x10² |
| | Reduction ratio(%) | 99.9 | 99.9 | 99.9 |
| Embodiment 2 | TCID₅₀ | 3.171.5x10² | 2.171.5x10² | 2.01.0x10² |
| | Reduction ratio(%) | 99.9 | 99.9 | 99.9 |

As a result of the tests, all of the embodiments show the excellent antiviral performances.

### Industrial Applicability

The air cleaning filter comprising the kimchi lactic acid bacteria and disinfectant coated thereon according to the present invention can clean up microbes, such as bacteria, fungi, and virus from the air by sterilizing the microbes, and can provide a safe and effective antiviral effect against the virus that causes the bird flu, the human influenza virus, and the new kind of influenza virus.

## Claims

1. An air cleaning filter for removing or sterilizing bacteria, fungi, or virus from the air, wherein the filter comprises a carrier coated with kimchi lactic acid bacteria and disinfectant, wherein the disinfectant is any one selected from a group including sodium desoxycholate, glutaraldehyde and quaternary ammonium, and wherein the shape of the filter is any one selected from a group including a honey comb shape, a granule shape, a net shape, a filter paper shape, a cotton shape, a mesh shape, a plate shape, and a foam shape,
**characterized in that**
the carrier further comprises a fermented *Ecklonia cava* extract.

2. The air cleaning filter as claimed in claim 1, wherein the kimchi lactic acid bacteria is included in the form of any one selected from a group including a Kimchi lactic acid bacteria culture solution, a concentration of the culture solution, an extract of the culture solution, a dried culture solution thereof, and a mixture thereof.

3. A method for producing an air cleaning filter of the claim 1 comprising the step of coating and immobilizing the kimchi lactic acid bacteria, the disinfectant and the fermented *Ecklonia cava* extract on the carrier, wherein the disinfectant is any one selected from a group including sodium desoxycholate, glutaraldehyde and quaternary ammonium,.

4. The method as claimed in claim 3, wherein the step of coating and immobilizing the kimchi lactic acid bacteria, the disinfectant and the fermented *Ecklonia cava* extract on the carrier includes the step of coating a coating solution including the kimchi lactic acid bacteria, the disinfectant, the fermented *Ecklonia cava* extract, and a binder selected from a group including silicon modified acryl resin, silicon modified epoxy resin, urethane resin, acryl resin, and silicon resin on the carrier, and the step of drying the carrier having the coating solution coated thereon thus.

5. The method as claimed in claim 4, wherein the coating solution further includes metal.

6. The method as claimed in claim 3, wherein the kimchi lactic acid bacteria is any one selected from a group including a Kimchi lactic acid bacteria culture solution, a concentration of the culture solution, an extract of the culture solution, a dried culture solution thereof, and a mixture thereof.

## Patentansprüche

1. Luftreinigungsfilter zur Entfernung oder Sterilisation von Bakterien, Pilzen oder Viren aus der Luft, wobei der Filter einen Träger aufweist, der mit Kimchi-Milchsäurebakterien und einem Desinfektionsmittel beschichtet ist, wobei das Desinfektionsmittel irgendeines ist, das aus einer Gruppe ausgewählt ist, die Natriumdesoxycholat, Glutaraldehyd und quaternäres Ammonium umfasst, und wobei die Form des Filters irgendeine ist, die aus einer Gruppe ausgewählt ist, die eine Honigwabenform, eine Granulatform, eine Netzform, eine Filterpapierform, eine Baumwollform, eine Gewebeform, eine Plattenform und eine Schaumform umfasst,
**dadurch gekennzeichnet, dass**
der Träger ferner einen fermentierten Ecklonia Cava Extrakt aufweist.

2. Luftreinigungsfilter nach Anspruch 1, wobei die Kimchi-Milchsäurebakterien in irgendeiner Form enthalten sind, die aus einer Gruppe ausgewählt ist, die eine Kimchi-Milchsäurebakterienkulturlösung, eine Konzentration der Kulturlösung, einen Extrakt der Kulturlösung, eine getrocknete Kulturlösung davon und eine Mischung davon umfasst.

3. Verfahren zum Herstellen eines Luftreinigungsfilter nach Anspruch 1, das den Schritt des Beschichtens und Immobilisierens der Kimchi-Milchsäurebakterien, des Desinfektionsmittels und des fermentierten Ecklonia Cava Extrakt auf dem Träger aufweist, wobei das Desinfektionsmittel irgendeines ist, das aus einer Gruppe ausgewählt ist, die Natriumdesoxycholat, Glutaraldehyd und quaternäres Ammonium umfasst.

4. Verfahren nach Anspruch 3, wobei der Schritt des Beschichtens und Immobilisierens der Kimchi-Milchsäurebakterien, des Desinfektionsmittels und des fermentierten Ecklonia Cava Extrakts auf dem Träger den Schritt des Beschichtens einer Beschichtungslösung, die die Kimchi-Milchsäurebakterien, das Desinfektionsmittel, den fermentierten Ecklonia Cava Extrakt und ein Bindemittel enthält, das aus einer Gruppe ausgewählt ist, die siliziummodifiziertes Acrylharz, siliziummodifiziertes Epoxidharz, Urethanharz, Acrylharz und Silikonharz umfasst, auf dem Träger, und den Schritt des Trocknens des Trägers umfasst, auf dem die Beschichtungslösung so beschichtet ist.

5. Verfahren nach Anspruch 4, wobei die Beschichtungslösung ferner ein Metall enthält.

6. Verfahren nach Anspruch 3, wobei die Kimchi-Milchsäurebakterien irgendwelche sind, die aus einer Gruppe ausgewählt sind, die eine Kimchi-Milchsäurebakterienkulturlösung, eine Konzentration der Kulturlösung, einen Extrakt der Kulturlösung, eine getrocknete Kulturlösung davon und eine Mischung davon umfasst.

## Revendications

1. Filtre purificateur d'air pour l'élimination ou la stérilisation de bactéries, de champignons ou de virus dans l'air, ledit filtre comprenant un support enduit de bactéries d'acide lactique de kimchi, et d'un désinfectant, ledit désinfectant étant sélectionné dans un groupe comprenant désoxycholate de sodium, glutaraldéhyde et ammonium quaternaire, et la forme du filtre étant sélectionnée dans un groupe comprenant une forme d'alvéole, une forme de granule, une forme de papier filtre, une forme de coton, une forme de maille, une forme de plaque et une forme de mousse, **caractérisé en ce que** le support comprend en outre un extrait d'*Ecklonia cava* fermenté.

2. Filtre purificateur d'air selon la revendication 1, où les bactéries d'acide lactique de kimchi sont présentées sous la forme sélectionnée dans un groupe comprenant une solution de culture de bactéries d'acide lactique de kimchi, une concentration de la solution de culture, un extrait de la solution de culture, une solution de culture séchée de celles-ci, et un mélange de ceux-ci.

3. Procédé de production d'un filtre purificateur d'air selon la revendication 1, comprenant l'étape d'enduction et d'immobilisation des bactéries d'acide lactique de kimchi, du désinfectant et de l'extrait d'*Ecklonia cava* fermenté sur le support, le désinfectant étant sélectionné dans un groupe comprenant désoxycholate de sodium, glutaraldéhyde et ammonium quaternaire.

4. Procédé selon la revendication 3, où l'étape d'enduction et d'immobilisation des bactéries d'acide lactique de kimchi, du désinfectant et de l'extrait d'*Ecklonia cava* fermenté sur le support comprend l'étape d'application d'une solution d'enduction comprenant les bactéries d'acide lactique de kimchi, le désinfectant, l'extrait d'*Ecklonia cava* fermenté, et un liant sélectionné dans un groupe comprenant une résine acrylique modifiée par de la silicone, une résine époxy modifiée par de la silicone, une résine uréthane, une résine acrylique et une résine de silicone sur le support, et l'étape de séchage du support avec la solution d'enduction ainsi appliquée.

5. Procédé selon la revendication 4, où la solution d'enduction comprend en outre un métal.

6. Procédé selon la revendication 3, où les bactéries d'acide lactique de kimchi sont sélectionnées dans un groupe comprenant une solution de culture de bactéries d'acide lactique de kimchi, une concentration de la solution de culture, un extrait de la solution de culture, une solution de culture séchée de celles-ci, et un mélange de ceux-ci.
